# EUROPEAN PATENT SPECIFICATION

(11) **EP 1 667 763 B1**
(45) Date of publication and mention of the grant of the patent: **05.09.2007**
(21) Application number: 04784279.4
(22) Date of filing: 15.09.2004
(51) Int. Cl.: A61N 1/05, C12N 15/63, C12N 15/87

(54) **DELIVERING GENETIC MATERIAL TO A STIMULATION SITE**
ABGABE VON GENETISCHEM MATIERAL AN EINE STIMULATIONSSTELLE
ACHEMINEMENT DE MATERIEL GENETIQUE VERS UN SITE DE STIMULATION

(30) Priority: 15.09.2003 US 663570
(43) Date of publication of application: 14.06.2006
(73) Proprietor: MEDTRONIC, INC., Minneapolis, Minnesota 55432-5604 (US)
(72) Inventor: MONGEON, Luc, R., Minneapolis, MN 55404 (US); CASAS-BEJAR, Jesus, Brooklyn Park, MN 55443 (US); MARKOWITZ, H., Toby, Roseville, MN 55113 (US); CROSS, Daisy, P., Minneapolis, MN 55418 (US); BLUM, Janelle, Saint Paul, MN 55117 (US); EBERT, Michael, Fridley, MN 55421 (US); LASKE, Timothy, G., Shoreview, MN 55126 (US)
(74) Representative: Hilleringmann, Jochen
(86) International application number: PCT/US2004/030364
(87) International publication number: WO 2005/028024

(56) References cited:
- WO-A-99/36563
- US-A- 5 265 608
- US-A- 5 702 384
- US-A1- 2002 012 914
- US-A1- 2003 073 238

## Description

### TECHNICAL FIELD

The invention relates to gene therapy and, more particularly, to delivery of genetic material to selected tissues to cause transgene expression by the selected tissues.

### BACKGROUND

A cardiac pacemaker delivers electrical stimuli, i.e., pacing pulses, to a heart to cause the heart depolarize and contract In general, pacemakers are provided to patients whose hearts are no longer able to provide an adequate or physiologically appropriate heart rate or contraction pattern. For example, patients who have been diagnosed as having bradycardia, or who have inadequate or sporadic atrio-ventricular (A-V) conduction may receive a pacemaker.

Cardiac pacemakers deliver pacing pulses to the heart via one or more electrodes. Typically, the electrodes are placed in contact with myocardial tissue to facilitate delivery of pacing pulses to the heart. The electrodes may be placed at endocardial or epicardial stimulation sites that are selected based on the pacing therapy that is to be provided to a patient.

Implanted cardiac pacemakers rely on a battery to provide energy for delivery of pacing pulses. Batteries of implanted pacemakers may be exhausted after several years of pacing. In general, when a battery of an implanted pacemaker is exhausted, the exhausted pacemaker must be explanted, and a new pacemaker implanted in its place. Consequently, in order to prolong the useful life of pacemakers, it is desirable to deliver pacing pulses at the lowest current or voltage amplitude that is still adequate to capture the heart.

Existing techniques for prolonging the life of pacemaker batteries include use of automatic capture threshold detection algorithms by pacemakers to maintain pacing pulse energy levels at the lowest level necessary for capture. Other existing techniques are directed toward reducing the pacing pulse energy level required to capture the heart. Such techniques include use of high impedance leads, and use of electrode designs that concentrate current in a small area in order to allow high current density at lower pacing pulse amplitudes. Electrodes that elute steroids or other anti-inflammatory agents have been developed to reduce inflammation and growth of fibrous tissue at the electrode/myocarditum interface, e.g. the stimulation site, which decreases the pacing pulse amplitude necessary to capture the heart.

WO-A-99/36563 discloses an electrode with injection means (syringe and needle). US-A-5 265 808 describes an electrode comprising polymeric material.

### SUMMARY

The invention provides an implantable medical lead according to claim 1 and a method of making the medical lead as defined in claim 15. The dependent claims relate to individual embodiments of the invention.

In general, the invention is directed to techniques for delivery of genetic material to tissue at a stimulation site, e.g., an electrode/tissue interface. Delivery of genetic material to a stimulation site causes transgene expression by tissue at the stimulation site. In some embodiments, the delivered genetic material causes increased expression of proteins, such as connexins, gap junctions, and ion channels, to increase the conductivity of the tissue at the stimulation site. In some embodiments, the delivered genetic material causes expression of a metalloproteinase, an anti-inflammatory agent, or an immunosuppressant agent.

Genetic material is delivered to the stimulation site via a stimulation lead. The stimulation lead includes a chamber that contains a matrix. The matrix absorbs the genetic material and elutes the genetic material to the stimulation site. The matrix is a polymeric matrix that in some embodiments includes collagen and takes the form of a sponge-like material. Cross-linking of the matrix controls the timing and rate of elution of genetic material from the matrix.

In one embodiment, the invention is directed to a method in which electrical stimulation is delivered to tissue of a patient at a stimulation site via an electrode mounted on a lead and located proximate to the stimulation site. The lead includes a chamber body that defines a chamber and the chamber contains a polymeric matrix. Genetic material is eluted from the matrix to the stimulation site to cause transgene expression by the tissue at the stimulation site. The genetic material may cause expression of a protein that increases the conductivity of the tissue at the stimulation site, such as connexin-43.

In another embodiment, the invention is directed to medical lead that comprises a lead body, an electrode mounted on a lead body to deliver electrical stimulation to the stimulation site, and a chamber body that defines a chamber. The chamber contains a polymeric matrix that absorbs the genetic material and elutes the genetic material to the tissue at the stimulation site. In some embodiments, the electrodes are porous to facilitate elution of the genetic material to the stimulation site.

In another embodiment, the invention is directed to a method in which a genetic material is introduced to a polymeric matrix, and the matrix is placed into a chamber formed by a chamber body of a medical lead for elution of the genetic material to tissue of a patient at a stimulation site. The method may further include blending extracellular collagen and gelatin to form the matrix.

The invention may provide advantages. For example, the transgene expression resulting from delivery of genetic material to a stimulation site may improve characteristics of the electrode tissue interface, such as the improvement of a sensing capability of the lead at this interface, or a reduction of the stimulation intensity necessary to achieve a desired effect. Specifically, transgene expression may result in increased tissue conductivity, reduced of fibrous growth, and/or reduced inflammation at the stimulation site. Furthermore, expression of a transgene may result in a desired effect that lasts longer and is more localized than that of drug.

Where the stimulation site is a cardiac site, transgene expression may result in a reduction in the pacing pulse amplitude necessary to capture the heart. In some cardiac pacing embodiments, tissue exhibiting increased conductivity may form a preferential conduction pathway to the specialized, intrinsic conduction system of the heart. Conduction of pacing pulses via such a pathway may lead to more synchronous, hemodymanically efficient contraction of the heart.

The details of one or more embodiments of the invention are set forth in the accompanying drawings and the description below. Other features, objects, and advantages of the invention will be apparent from the description and drawings, and from the claims.

### BRIEF DESCRIPTION OF DRAWINGS

FIG. 1 is a conceptual diagram illustrating an exemplary environment in which genetic material is delivered to a stimulation site.

FIG. 2 is a conceptual diagram illustrating the environment of FIG. 1 in greater detail.

FIGS. 3A and 3B are cross-sectional diagrams illustrating an example medical lead that delivers genetic material to a stimulation site.

FIG. 4 is a flowchart illustrating an example method for delivery of genetic material to a stimulation site using a medical lead.

FIG. 5 is a flowchart illustrating an example method for providing a medical lead that includes genetic material.

### DETAILED DESCRIPTION

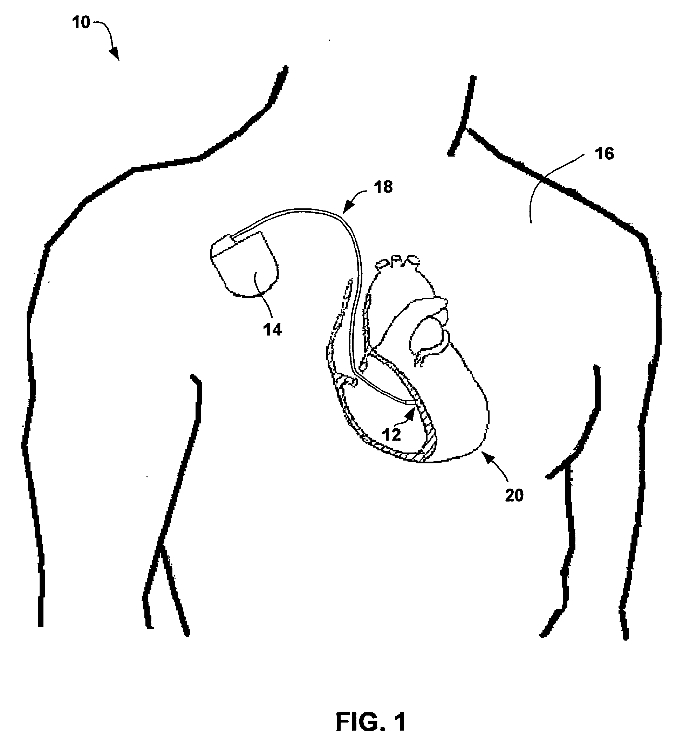

FIG. 1 is a conceptual diagram illustrating an exemplary environment 10 in which genetic material is delivered to a stimulation site 12. In the illustrated environment 10, an implantable pulse generator (IPG) 14 delivers electrical stimulation to tissue of a patient 16 at stimulation site 12 via a lead 18. As shown in FIG. 1, IPG 14 may take the form of an implanted cardiac pacemaker or pacemaker-cardioverter-defibrillator, and deliver electrical stimulation in the form of pacing pulses, cardioversion pulses, or defibrillation pulses to the heart 20 of patient 16. Although illustrated in FIG. 1 as coupled to a single lead 18 to deliver pacing pulses to a single endocardial stimulation site 12, IPG 14 may be coupled to any number of leads 18 and deliver pacing pulses to any number of endocardial or epicardial stimulation sites.

As will be described in greater detail below, genetic material is delivered to stimulation site 12 via lead 18. The genetic material is delivered, for example, via a viral vector, such as an adenoviral or adeno-associated viral vector. Additionally, or alternatively, the genetic material is delivered via a liposomal vector, or as plasmid deoxyribonucleic acid (DNA).

The delivered genetic material causes transgene expression by the tissue located at stimulation site 12, which may, in turn, reduce the pacing pulse amplitude necessary to capture heart 20 and consequently prolong the life of a battery used by IPG 14 as a source of energy for delivery of pacing pulses to heart 20. In some embodiments, the delivered genetic material causes increased expression of connexins, gap-junctions, ion channels, or the like by the tissue at stimulation site 12, which, in turn, increases the conductivity of the tissue at stimulation site 12. An exemplary protein which may be expressed to increase the conductivity of the tissue at stimulation site 12 is connexin-43. Tissue exhibiting increased conductivity at stimulation site 12 forms a virtual biological electrode in contact with an electrode located on lead 18, and delivery of pacing pulses from the electrode located on lead 18 to the virtual biological electrode at stimulation site 12 may facilitate capture of heart 20 at lower pacing pulse amplitudes.

In some embodiments, the delivered genetic material causes expression of metalloproteinases, or anti-inflammatory or immunosuppressant agents, which effect extracellular matrix physiology and/or remodeling and may reduce fibrous growth and/or inflammation at stimulation site 12. An exemplary anti-inflammatory agent that may be expressed is IκB, or other anti-inflammatory mediators of the NF-κB cascade. Reduced fibrous growth and/or inflammation at the stimulation site leads to a reduction in the pacing pulse amplitude necessary to capture heart 20.

In some embodiments, two or more genetic materials are delivered to stimulation site 12. Drugs, such as dexamethasone, may also be delivered to stimulation site 12. Various genetic materials and drugs can be delivered to stimulation site 12 simultaneously, or in a predetermined order. In exemplary embodiments, the timing and duration of delivery of each type of genetic material or drug is controlled, as will be described in greater detail below.

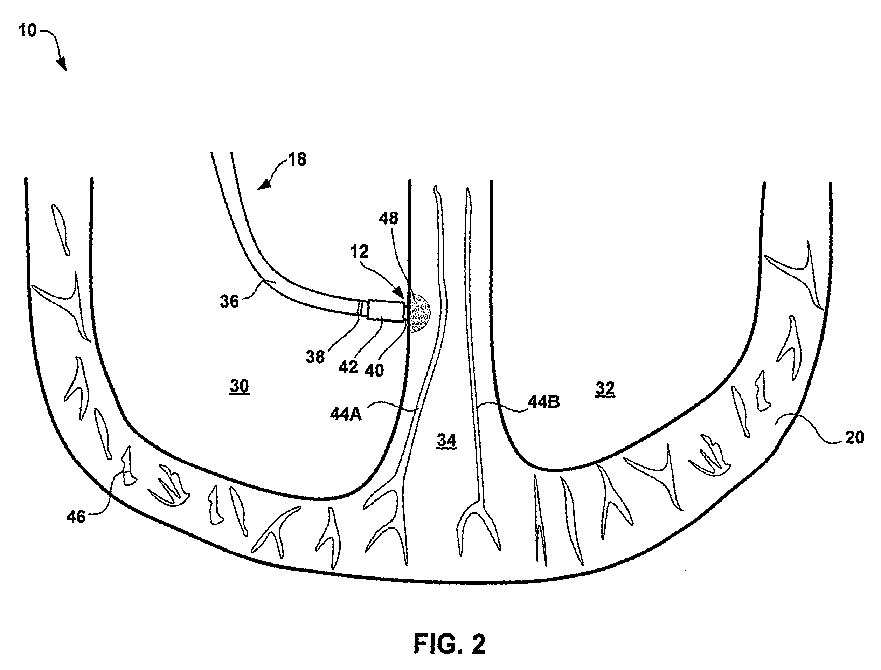

FIG. 2 is a conceptual diagram illustrating environment 10 in greater detail. The right ventricle 30 and left ventricle 32 of heart 20 are shown in FIG. 2. In the illustrated example, lead 18 extends from IPG 14 (FIG. 1), through blood vessels (not shown) of patient 16, to stimulation site 12 within right ventricle 30. In the illustrated example, stimulation site 12 is located on the intraventricular septum 34 of heart 20.

Lead 18 is a bipolar pace/sense lead. Lead 18 includes an elongated insulated lead body 36 carrying a number of concentric coiled conductors (not shown) separated from one another by tubular insulative sheaths (not shown). Located adjacent to the distal end of lead 18 are bipolar electrodes 38 and 40. Electrode 38 may take the form of a ring electrode, and electrode 40 may take the form of an extendable helix tip electrode mounted retractably within an insulated electrode head 42. Each of the electrodes 38 and 40 is coupled to one of the coiled conductors within lead body 36.

FIG. 2 also illustrates a portion of the intrinsic specialized conduction system of heart 20, which includes bundles of His 44A and 44B (collectively "bundles of His 44"), and Purkinje fibers 46. For ease of illustration, only a single Purkinje fiber 46 is labeled in FIG. 2. Bundles of His 44 and Purkinje fibers 46 are made up of cells that are more conductive than the non-specialized myocardial cells that form much of heart 20. Intrinsic depolarizations of heart 20 originating in the atria (not shown) are rapidly conducted from an atrio-ventricular node (not shown) throughout ventricles 30 and 32 by bundles of His 44 and Purkinje fibers 46. This rapid conduction enabled by bundles of His 44 and Purkinje fibers 46 leads to a coordinated and hemodynamically effective contraction of ventricles 30 and 32. Typically, pacing pulses are delivered to non-specialized myocardial tissue, and do not provide a contraction that is as coordinated or hemodynamically effective as that achieved through use of bundles of His 44 and Purkinje fibers 46.

As illustrated in FIG. 2, delivery of genetic material to stimulation site 12 causes transgene expression by a region of tissue 48 proximate to stimulation site 12. In some embodiments, as described above, the transgene expression by tissue 48 leads to increased conductivity of tissue 48. Further, in some embodiments, region 48 may extend to bundle of His 44A. In such embodiments, tissue 48 with increased conductivity forms a preferential conduction pathway from electrode 40 to the specialized conduction system of heart 20. Pacing pulses delivered to stimulation site 12 may be rapidly conducted by tissue 48 to bundle of His 44A, and from bundle of His 44A throughout ventricles 30 and 32 by the specialized conduction system of heart, leading to more coordinated and hemodynamically effective contractions than may be achieved by delivery of pacing pulses without delivery of genetic material to stimulation site 12.

The location of lead 18 and stimulation site 12 illustrated in FIG. 2 is merely exemplary. For example, stimulation site 12 may be located at any point within ventricles 30 and 32, or epicardially on ventricles 30 and 32, and tissue 48 may form a preferential conduction pathway to either of bundles of His 44 or any of Purkinje fibers 46. Further, stimulation site 12 may be located endocardially or epicardial at either of the atria of heart 20. Moreover, as described above with reference to FIG. 1, tissue 48 need not form a preferential conduction pathway, nor is transgene expression by tissue 48 limited to transgene expression that increases the conductivity of tissue 48.

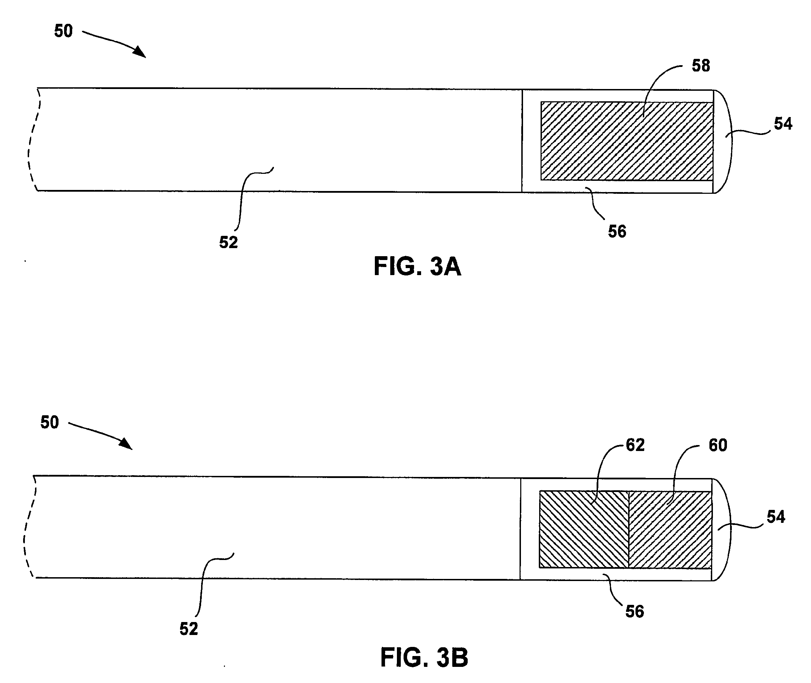

FIGS. 3A and 3B are cross-sectional diagrams illustrating an example medical lead 50 that delivers genetic material to a stimulation site 12. Lead 50 includes a lead body 52 and an electrode 54. Like lead 18 illustrated in FIGS. 1 and 2, lead 50 may be a bipolar pace/sense lead. However, for ease of illustration, only single electrode 54 of lead 50 is depicted in FIGS. 3A and 3B.

As shown in FIGS. 3A and 3B, the distal portion of lead 50 includes a chamber body 56 that contains genetic material for delivery to stimulation site 12. In some embodiments, chamber body 56 is in fluid communication with electrode 54, and electrode 54 is porous, or may be otherwise formed to facilitate elution of genetic material from chamber body 56 to stimulation site 12.

Although illustrated in FIGS. 3A and 3B as a hemispherical shape, an exemplary electrode has a helical shape or is otherwise configured as is known in the art to allow fixation of electrode 54 at stimulation site 12. Electrode 54 may be made of sintered carbon or other materials known in the art. In some embodiments, chamber body 56 includes an electrically conductive element (not shown) or is constructed, at least in part, from an electrically conductive material, to allow conduction of pacing pulses to electrode 54.

As shown in FIG. 3A, chamber body 56 contains a matrix 58 to hold and preserve the genetic material for delivery to stimulation site 12. Matrix 58 is a polymeric matrix, and may take the form of a sponge-like material that absorbs the genetic material, and degrades to elute the genetic material to stimulation site 12 via electrode 54. In an exemplary construction, matrix 58 includes extracellular collagen.

In some embodiments, matrix 58 is designed, based on the one or more genetic materials selected to be delivered to stimulation site 12, to provide the desired timing and rate of release of the selected genetic materials that will provide adequate transfection efficiency for the selected genetic materials. The timing and rate of release of genetic materials to stimulation site 12 is a function of the degradation rate of matrix 58, which may be controlled by the extent of cross-linking of matrix 58.

As described above, two or more genetic materials, or in some embodiments at least one genetic material and one or more drugs, may be delivered to stimulation site 12. The genetic materials and drugs may be delivered, for example, simultaneously as a mixture, or in a predetermined staged sequence. In general, matrix 58 will degrade from electrode 54 toward lead body 52. Consequently, where chamber body 56 includes a single matrix 58, as illustrated in FIG. 3A, the timing of delivery of the various genetic materials and drugs is controlled based on the position of the genetic materials and drugs within matrix 58.

In some embodiments, as shown in FIG. 3B, chamber body 56 includes two or more matrices 60 and 62. Each of matrices 60 and 62 may include one or more genetic materials and one or more drugs. The timing of delivery of genetic materials and drugs is controlled by the position of their respective matrices along the main axis of lead 50. The duration of delivery of genetic materials and drugs is controlled by the cross-linking and size of their respective matrices. A chamber body 56 according to the invention may include any number of matrices arranged in any manner.

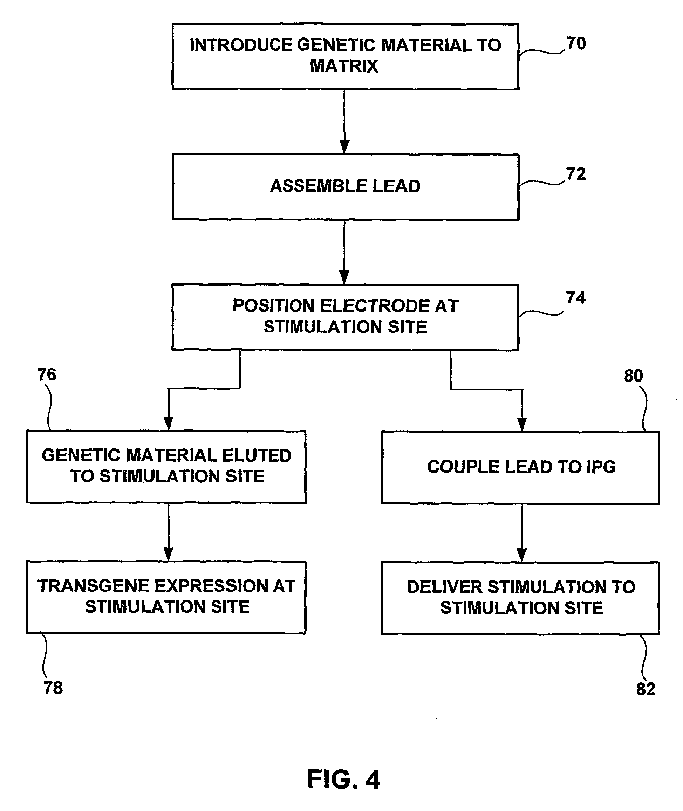

FIG. 4 is a flowchart illustrating an example method for delivery of genetic material to stimulation site 12 using a medical lead 50 (FIG. 3A). Genetic material is introduced to matrix 58 (70). For example, where matrix 58 takes the form of a polymeric sponge, matrix 58 is soaked in or injected with the genetic material. Chamber body 56 may be separable from lead 50 to allow access to chamber body so that matrix 58 including the genetic material may be placed in chamber body.

Prior to implantation in patient 16, lead 50 is assembled (72). In some embodiments, a manufacturer of lead 50 introduces genetic material into matrix 58 and inserts matrix 58 into chamber body 56. Chamber body 56 containing matrix 58 is frozen to preserve the genetic material during delivery of the components of lead 50 to the clinician. Prior to implantation of lead 50 into patient 16, the clinician thaws chamber body 56, and assembles lead 50. Alternatively, lead 50 is preassembled, and the assembled lead 50 is frozen for storage and delivery to the clinician. In still other embodiments, prior to implantation of lead 50 into patient 16, the clinician introduces the genetic material into matrix 58, inserts matrix 58 into chamber body 56, and assembles lead 50, or immerses the distal end of a previously assembled lead 50 into the genetic material.

When implanting lead 50 into patient 16, the clinician positions electrode 54 at stimulation site 12 (74), and couples a proximal end of lead 50 to IPG 14 (76). IPG 14 delivers stimulation in the form of pacing pulses to stimulation site 12 via lead 50 and electrode 54 (78). When electrode 54 is positioned at stimulation site 12, the genetic material is eluted from matrix 58, through electrode 54, to tissue 48 at stimulation site 12 (80). The eluted genetic material causes transgene expression by tissue 44 at stimulation site 12 (82).

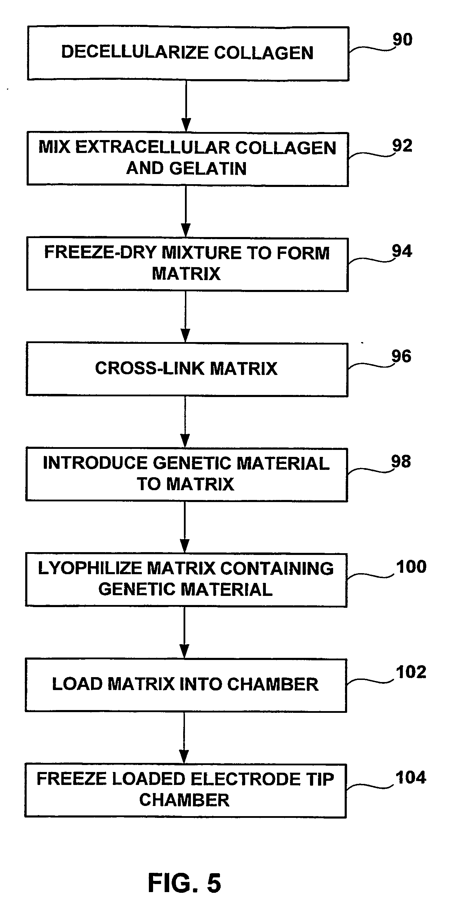

FIG. 5 is a flowchart illustrating an example method for providing medical lead 50 that includes genetic material. In particular, FIG. 5 illustrates a method that includes creation of a polymeric matrix 58 formed from extracellular collagen. Collagen is decellularized (90), and mixed with gelatin (92). For example, a 5% weight to volume (w/v) solution of extracellular collagen may be blended with a 5% (w/v) solution of gelatin. The resulting mixture may be poured into a form, and is freeze-dried to form matrix 58, which in exemplary embodiments takes the form of a sponge (94).

Resulting matrix 58 is cross-linked (96). Exemplary methods for cross-linking collagen matrices include immersion in a 0.5% (w/v) solution of diphenylphosphorylazide (DPPA) in dimethylformamide (DMF), a 0.05% (w/v) solution of glutaradehyde (GTA), or a 0.05 Molar (M) solution of N-(3-Dimethylaminopropyl)-N'-etheylcarbodiimide (EDC) and N-hydroxysuccinimide (NHS). As described above, the cross-linking of matrix 58 affects the elution rate of genetic material stored therein.

Genetic material is introduced into matrix 58 (98), and matrix 58 is lyophilized (100) in the presence of a lyophilization stabilizer. As an example, a 0.5 M sucrose solution may be used to stabilize gene complexes within the matrix 58 during the process of lyophilization. Matrix 58 is loaded into chamber body 56 (102), and chamber body 56 is frozen for storage and delivery to a clinician (104). Chamber body 56 containing matrix 58, or the entire lead 50, is stored, for example, at -70° C.

The following examples are meant to be exemplary of embodiments of the invention, and are not meant to be limiting.

Example 1 - DPPA Crosslinking of Collagen/Gelatin Matrix.

The matrix is immersed in a 0.5% (w/v) solution of DPPA in DMF at 4° C for twenty-four hours. The matrix is then rinsed in a borate buffer three times, for ten to fifteen minutes per rinse, using approximately 50 mls of the borate buffer for each rinse. The borate buffer includes 0.04 M each of boric acid and Borax. The matrix is then incubated overnight at 4° C in the borate buffer, and rinsed three times in a 70% ethanol solution, using approximately 50 mls of the ethanol solution per rinse.

Example 2 - GTA Crosslinking of Collagen/Gelatin Matrix.

The matrix is incubated for one hour at room temperature in a freshly made 0.05% (w/v) GTA solution. The matrix is then washed in a 0.1 M glycine (pH 7.4) solution for one hour at room temperature using approximately 50 ml of glycine solution.

Example 3 - EDC/NHS Crosslinking of Collagen/Gelatin Matrix.

Matrix is washed in a 0.05 M solution of 2-moephdinoethane sulfonic acid (MES) for about thirty minutes (~50 mls). The matrix is then immersed in a 0.05 M solution of EDC and NHS in the MES buffer, shaken gently, and incubated for four hours. The matrix is then washed is a 0.1 M solution of dibasic sodium phosphate for two hours using approximately 50 mls of the solution. Following the sodium phosphate wash, the matrix is washed four times in deionized water, for thirty minutes and using 50 mls of deionized water per wash.

Various embodiments of the invention have been described. However, one skilled in the art will appreciate that various modifications can be made to the described embodiments. For example although the invention has been described herein in the context of cardiac pacing, stimulation sites may be located, and genetic material may be delivered to tissues, anywhere within or on the surface of a patient. The invention may be applied in the context of, for example, neurostimulation, muscular stimulation, gastrointestinal stimulation, and bladder stimulation. Leads may be, for example, implanted leads, percutaneous leads, or external leads that provide transcutaneous stimulation. Electrodes may be, for example, bipolar or unipolar pacing electrodes, multiple electrode arrays used for neurostimulation, coil electrodes used for defibrillation or cardioversion, patch electrodes, or cuff electrodes.

## Claims

1. An implantable medical lead comprising:
- a lead body (36;52),
- an electrode (38,40;54) mounted on the lead body (36;52) to deliver electrical stimulation to a stimulation site (12), and
- a chamber body (56) that defines a chamber, the chamber containing a polymeric matrix (58,60,62) adapted to absorb genetic material and elute the genetic material to tissue at the stimulation site (12).

2. The implantable medical lead of claim 1, wherein the matrix (58,60,62) comprises extracellular collagen.

3. The implantable medical lead of claim 1 or 2, wherein the matrix (58,60, 62) is cross-linked, and elutes the absorbed genetic material at a rate that is a function of the cross-linking.

4. The implantable medical lead of any one of claims 1 to 3, wherein the chamber body (56) is separable from the lead for loading with the matrix (58,60,62) and the genetic material.

5. The implantable medical lead of any one of claims 1 to 4, wherein the electrode (38,40;54) is porous, and the matrix (58,60,62) elutes the genetic material to the stimulation site (12) via the electrode (38,40;54).

6. The implantable medical lead of any one of claims 1 to 5, wherein the genetic material comprises at least one of a viral vector, a liposomal vector, and plasmid deoxyribonucleic acid (DNA).

7. The implantable medical lead of any one of claims 1 to 6, wherein the genetic material causes expression of a protein by the tissue at the stimulation site (12) that increases the conductivity of the tissue at the stimulation site (12).

8. The implantable medical lead of claim 7, wherein the genetic material causes expression of at least one of a connexin, a gap-junction, and an ion channel by the tissue at the stimulation site (12).

9. The implantable medical lead of claim 8, wherein the genetic material causes expression of connexin-43 by the tissue at the stimulation site (12).

10. The implantable medical lead of any one of claims 1 to 5, wherein the genetic material causes expression of at least one of a metalloproteinase, an anti-inflammatory agent, and an immunosuppressant agent.

11. The implantable medical lead of claim 10, wherein the genetic material causes expression of 1κB.

12. The implantable medical lead of any one of claims 1 to 11, wherein the electrode (38,40; 54) is implantable within a patient.

13. The implantable medical lead of claim 12, wherein the tissue at the stimulation site (12) comprises cardiac tissue.

14. The implantable medical lead of claim 13, wherein the transgene expression in response to delivery of the genetic material creates a preferential conduction pathway between the stimulation site (12) and an intrinsic conduction system of a heart of the patient.

15. A method of making the implantable medical lead of any one of claims 1 to 14, comprising:
- introducing genetic material to a polymeric matrix (58,60,62), and
- placing the matrix (58) into a chamber formed by a chamber body (56) of an implantable medical lead (18) that includes an electrode (48,40; 54)

16. The method of claim 15, further comprising:
- blending extracellular collagen and gelatin, and
- freeze-drying the blended extracellular collagen and gelatin to from the matrix.

17. The method of claim 15 or 16, further comprising:
- identifying the genetic material and an elution rate, and
- cross-linking the matrix (58,60,62) based on the genetic material and the elution rate.

18. The method of any one of claims 15 to 17, further comprising lyophilizing the matrix (58,60,62) containing the genetic material.

## Patentansprüche

1. Implantierbare medizinische Leitung mit:
- einem Leitungskörper (36; 52),
- einer Elektrode (38, 40; 54), die an dem Leitungskörper (36; 52) angebracht ist, um elektrische Stimulation an einen Stimulationsort (12) auszugeben, und
- einem Kammerkörper (56), der eine Kammer bildet, welche eine Polymermatrix (58, 60, 62) enthält, die zum Absorbieren von genetischem Material und zum Eluieren des genetischen Materials zu Gewebe an dem Stimulationsort (12) geeignet ist.

2. Implantierbare medizinische Leitung nach Anspruch 1, bei welcher die Matrix (58, 60, 62) extrazellulares Kollagen aufweist.

3. Implantierbare medizinische Leitung nach Anspruch 1 oder 2, bei welcher die Matrix (58, 60, 62) vernetzt ist und das absorbierte genetische Material mit einer Rate eluiert, die eine Funktion der Vernetzung ist.

4. Implantierbare medizinische Leitung nach einem der Ansprüche 1 bis 3, bei welcher der Kammerkörper (56) zum Befüllen mit der Matrix (58, 60, 62) und dem genetischen Material von der Leitung trennbar ist.

5. Implantierbare medizinische Leitung nach einem der Ansprüche 1 bis 4, bei der die Elektrode (38, 40; 54) porös ist und die Matrix (58, 60, 62) das genetische Material über die Elektrode (38, 40; 54) an den Stimulationsort (12) eluiert.

6. Implantierbare medizinische Leitung nach einem der Ansprüche 1 bis 5, bei welcher das genetische Material mindestens eines der folgenden Bestandteile enthält: einen viralen Vektor, einen liposomalen Vektor, und Plasmid Desoxyribonukleinsäure (DNA).

7. Implantierbare medizinische Leitung nach einem der Ansprüche 1 bis 6, bei dem das genetische Material die Expression eines Proteins durch das Gewebe an dem Stimulationsort (12) bewirkt, wodurch die Leitfähigkeit des Gewebes an dem Stimulationsort (12) erhöht wird.

8. Implantierbare medizinische Leitung nach Anspruch 7, bei welcher das genetische Material die Expression mindestens eines Connexins und/oder einer Gap-Junction und/oder eines Ionenkanals durch das Gewebe am Stimulationsort (12) bewirkt.

9. Implantierbare medizinische Leitung nach Anspruch 8, bei welcher das genetische Material die Expression von Connexin-43 durch das Gewebe am Stimulationsort (12) bewirkt.

10. Implantierbare medizinische Leitung nach einem der Ansprüche 1 bis 5, bei welcher das genetische Material die Expression mindestens einer Metalloproteinase und/oder eines entzündungshemmenden Mittels und/oder eines Immunosuppressivums bewirkt.

11. Implantierbare medizinische Leitung nach Anspruch 10, bei welcher das genetische Material die Expression von 1κB bewirkt.

12. Implantierbare medizinische Leitung nach einem der Ansprüche 1 bis 11, bei welcher die Elektrode (38, 450; 54) in einen Patienten implantierbar ist.

13. Implantierbare medizinische Leitung nach Anspruch 12, bei welcher das Gewebe an dem Stimulationsort (12) Kardialgewebe umfasst.

14. Implantierbare medizinische Leitung nach Anspruch 13, bei welcher die transgene Expression in Reaktion auf die Ausgabe des genetischen Materials einen bevorzugten Leitungsweg zwischen dem Stimulationsort (12) und einem intrinsischen Leitungssystem des Herzens eines Patienten bildet.

15. Verfahren zur Herstellung der implantierbaren medizinischen Leitung nach einem der Ansprüche 1 bis 14, mit den folgenden Schritten:
- Einbringen von genetischem Material in eine Polymermatrix (58, 60, 62), und
- Anordnen der Matrix (58) in einer Kammer, die durch einen Kammerkörper (56) einer implantierbaren medizinischen Leitung (18) gebildet ist, welche eine Elektrode (38, 40; 54) aufweist.

16. Verfahren nach Anspruch 15, ferner mit den Schritten
- des Mischens von extrazellularem Kollagen und Gelatine; und
- des Gefriertrocknens der Mischung aus extrazellularem Kollagen und Gelatine zur Bildung der Matrix.

17. Verfahren nach Anspruch 15 oder 16, ferner mit den Schritten
- des Identifizierens des genetischen Materials und der Eluierungsrate, und
- des Vernetzens der Matrix (58, 60, 62) basierend auf dem genetischen Material und der Eluierungsrate.

18. Verfahren nach einem der Ansprüche 15 bis 17, ferner mit dem Schritt des Lyophilisierens der das genetische Material enthaltenden Matrix (58, 60, 62).

## Revendications

1. Conducteur médical implantable, comprenant :
- un corps de conducteur (36 ; 52) ;
- une électrode (38, 40 ; 54) montée sur le corps de conducteur (36 ; 52) pour acheminer une stimulation électrique vers un site de stimulation (12), et
- un corps de chambre (56) qui définit une chambre, la chambre contenant une matrice polymère (58, 60, 62) adaptée pour absorber un matériau génétique et éluer le matériau génétique en un tissu au site de stimulation (12).

2. Conducteur médical implantable selon la revendication 1, dans lequel la matrice (58, 60, 62) comprend du collagène extracellulaire.

3. Conducteur médical implantable selon la revendication 1 ou 2, dans lequel la matrice (58, 60, 62) est réticulée et élue le matériau génétique absorbé à une vitesse qui est une fonction de la réticulation.

4. Conducteur médical implantable selon l'une quelconque des revendications 1 à 3, dans lequel le corps de chambre (56) peut être séparé du conducteur pour le charger de la matrice (58, 60, 62) et du matériau génétique.

5. Conducteur médical implantable selon l'une quelconque des revendications 1 à 4, dans lequel l'électrode (38, 40 ; 54) est poreuse, et la matrice (58, 60, 62) élue le matériau génétique au site de stimulation (12) par l'intermédiaire de l'électrode (38, 40 ; 54).

6. Conducteur médical implantable selon l'une quelconque des revendications 1 à 5, dans lequel le matériau génétique comprend au moins un vecteur viral ou un vecteur liposomique, et un acide désoxyribonucléique (ADN) de plasmide.

7. Conducteur médical implantable selon l'une quelconque des revendications 1 à 6, dans lequel le matériau génétique provoque l'expression d'une protéine par le tissu au site de stimulation (12) qui accroît la conductivité du tissu au site de stimulation (12).

8. Conducteur médical implantable selon la revendication 7, dans lequel le matériau génétique provoque l'expression d'au moins une connexine ou une jonction de brèche ou un canal ionique par le tissu au site de stimulation (12).

9. Conducteur médical implantable selon la revendication 8, dans lequel le matériau génétique provoque l'expression de connexine-43 par le tissu au site de stimulation (12).

10. Conducteur médical implantable selon l'une quelconque des revendications 1 à 5, dans lequel le matériau génétique provoque l'expression d'au moins une métalloprotéinase ou un agent anti-inflammatoire ou un agent immunosuppresseur.

11. Conducteur médical implantable selon la revendication 10, dans lequel le matériau génétique provoque l'expression de 1κB.

12. Conducteur médical implantable selon l'une quelconque des revendications 1 à 11, dans lequel l'électrode (38, 40 ; 54) est implantable dans le corps d'un patient.

13. Conducteur médical implantable selon la revendication 12. dans lequel le tissu au site de stimulation (12) comprend un tissu cardiaque.

14. Conducteur médical implantable selon la revendication 13, dans lequel l'expression transgénique en réponse à l'acheminement du matériau génétique crée une voie de conduction préférentielle entre le site de stimulation (12) et un système de conduction intrinsèque du coeur d'un patient.

15. Procédé de fabrication du conducteur médical implantable selon l'une quelconque des revendications 1 à 14, comprenant :
- l'introduction d'un matériau génétique dans une matrice polymère (58, 60, 62), et
- la mise en place de la matrice (58) dans une chambre formée par un corps de chambre (56) d'un conducteur médical implantable (18) qui comprend une électrode (48, 40 ; 54).

16. Procédé selon la revendication 15, comprenant en outre:
- le mélange de collagène extracellulaire et de gélatine, et
- la lyophilisation du collagène extracellulaire et de la gélatine mélangés pour former la matrice.

17. Procédé selon la revendication 15 ou 16, comprenant en outre:
- l'identification du matériau génétique et de la vitesse d'élution, et
- la réticulation de la matrice (58, 60, 62) sur la base du matériau génétique et de la vitesse d'élution.

18. Procédé selon l'une quelconque des revendications 15 à 17, comprenant en outre la lyophilisation de la matrice (58, 60, 62) contenant le matériau génétique.
